# EUROPEAN PATENT APPLICATION

(11) **EP 2 573 089 A1**
(43) Date of publication of application: **27.03.2013**
(21) Application number: 11182536.0
(22) Date of filing: 23.09.2011
(51) Int. Cl.: C07D 493/18, A61K 31/357, A61P 33/06

(54) **Novel artmisinin derivatives**

(71) Applicant: Spago Imaging AB, 114 46 Stockholm (SE)
(72) Inventor: Axelsson, Oskar, 243 32 Höör (SE); Ek, Fredrik, 226 49 Lund (SE)
(74) Representative: Stenbäck, Maria Elisabeth

(57) **Abstract**

Compound of general formula **1** 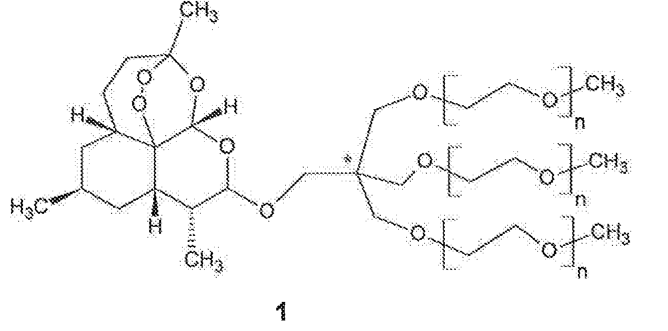 wherein n=1-5 are disclosed, as well as composition, such as pharmaceutical compositions, comprising such compounds, and use thereof in treatment of diseases such as malaria. Also methods for producing such compounds are disclosed.

## Description

The present invention relates to derivatives of the malaria drug artemisinin (also known as qinghaosu) with advantages over currently available derivatives. Important advantages are high water solubility, acid resistance, and high activity against the malaria parasites of the family *Plasmodium,* especially *P. falciparum.*

### Background

Artemisinin and its analogs are today the first line of treatment against severe malaria. Severe malaria is caused by the mosquito borne blood parasite Plasmodium falciparum. The compounds are highly active, curing the disease within a few hours. Currently artemisinin (a), dihydroartemisinin (b), artemether (c), and artesunate (d) are available for clinical use, often in combination with an additional active ingredient in so called ACT (Artemisinin-based Combination Therapy). Although highly successful drugs, the known compounds suffers from problems with solubility, bioavailability and short plasma half life. In particular is the short plasma half-life problematic because it can facilitate the development of resistant strains of the parasites.

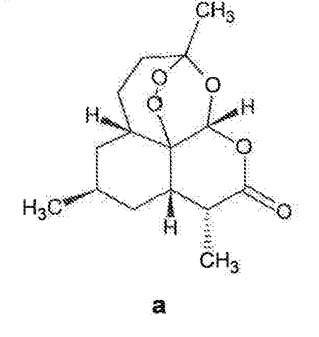

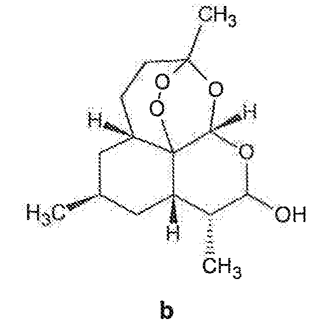

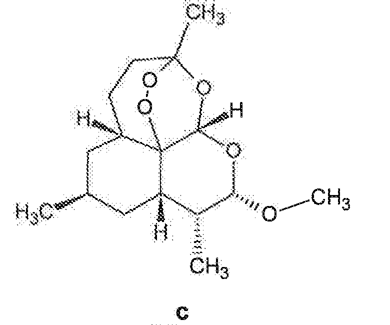

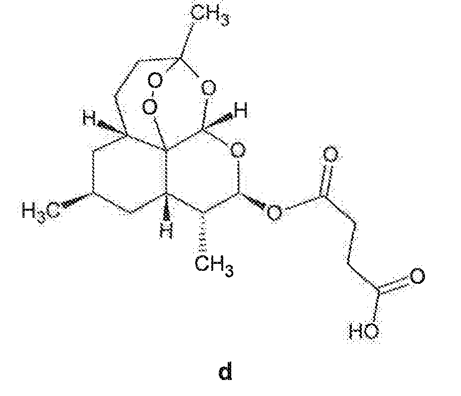

There are known several derivatives of artermsinin from the literature, but none that contain a branched polyether

Artesunate (d) is a compound with somewhat improved water solubility over the free Artemisinin (a). However, it suffers from low stability to acid and hence moderate bioavailability and a short shelf life .

### Definitions of terms

"Organic residue" refers to an organic compounds covalently bond to a molecular entity.

"Hydrocarbon" or "hydrocarbon chain" is an organic residue consisting of hydrogen and carbon. The hydrocarbon may be fully saturated or it may comprise one or more unsaturations. Unless otherwise specified, the hydrocarbon may contain any number of carbon atoms between 1 and 50. The hydrocarbon group of the compounds may then be designated for example as "C₁₋₈ hydrocarbon" for a hydrocarbon comprising 1, 2, 3, 4, 5, 6, 7 or 8 carbon atoms, or similar designations. Typical hydrocarbon groups include, but are in no way limited to, methyl, ethyl, propyl, isopropyl, butyl, isobutyl, tertiary butyl, pentyl, hexyl, ethenyl, propenyl, butenyl, phenyl, benzyl.

"alkyl" refers to a straight or branched hydrocarbon chain fully saturated (no double or triple bonds) hydrocarbon group. The alkyl group may have 1, 2, 3, 4, 5, 6,7 or 8 carbon atoms. The alkyl group of the compounds may be designated as "C₁₋₈ alkyl" or similar designations. Typical alkyl groups include, but are in no way limited to, methyl, ethyl, propyl, isopropyl, butyl, isobutyl, tertiary butyl, pentyl, hexyl, and the like.

Whenever it appears herein, a numerical range such as "1 to 8" or "1-8" refer to each integer in the given range; e.g., "1 to 8 carbon atoms" means that the alkyl group may consist of 1 carbon atom, 2 carbon atoms, 3 carbon atoms, etc., up to and including 8 carbon atoms. It is also implied that all sub-intervals are included e. g. 1-2, 1-5, 1-8, 2-3, 2-4, 2-8 and 3-5.

As used herein, "alkoxy" refers to the formula -OR wherein R is a C₁₋₈ alkyl, e.g. methoxy, ethoxy, n-propoxy, 1-methylethoxy (isopropoxy), n-butoxy, iso-butoxy, sec-butoxy, tert-butoxy, amyloxy, iso-amyloxy and the like. An alkoxy may be optionally substituted.

As used herein, "heterocycle" refers to a stable 3- to 18 membered ring which consists of carbon atoms and from one to five heteroatoms selected from the group consisting of nitrogen, oxygen and sulfur. The heterocycle may be monocyclic, bicyclic or tricyclic.

"Strong base" refers in the current context to bases that are stronger than hydroxide and not compatible with aqueous environments.

### Summary

The present disclosure relates to novel polyether compounds. The problems associated with artemisinin and its analogs used today in treatment of malaria are overcome or reduced with these novel polyether compounds.

As mentioned above, there are known several derivatives of artemisinin from the literature. None of these contains a branched polyether. Whithout being bound by any theory, the fact that the compound of the present disclosure contains a branched polyeter may what results in the advantages associated with these novel compounds.

Disclosed are compounds of general formula 1 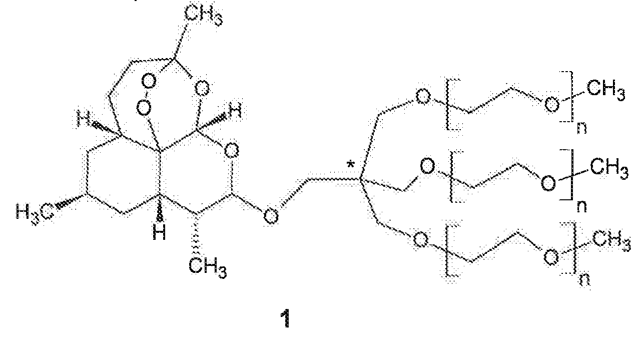 wherein n=1-5.

Also compositions comprising the above compounds are disclosed.

Furthermore, pharmaceutical compositions comprising the above compounds are disclosed. Such compositions comprises at least one compound as disclosed herein in combination with at least one pharmaceutically acceptable adjuvant or carrier. Such a composition may be formulated as a tablet, a capsule, a lozenge, a liquid suitable for oral ingestion, a reconstitutable solid suitable for reconstitution with water or saline to a solution for injection or infusion, or a solution ready for injection or infusion. Such a composition may further comprise at least one additional drug, such as an antimalarial drug.

The compounds and/or pharmaceutical compositions may be used in the treatment of malaria.

The compounds may also be used in the production of a pharmaceutical composition for use in the treatment of malaria.

The compounds and/or pharmaceutical compositions may also be used in a method of treatment of malaria, wherein a pharmaceutically active amount of a comound as disclosed herein is administered to a patient in need of such treatment.

Also processes for the production of the compounds disclosed herein are described.

### Detailed description

### First aspect - Compounds with formula 1

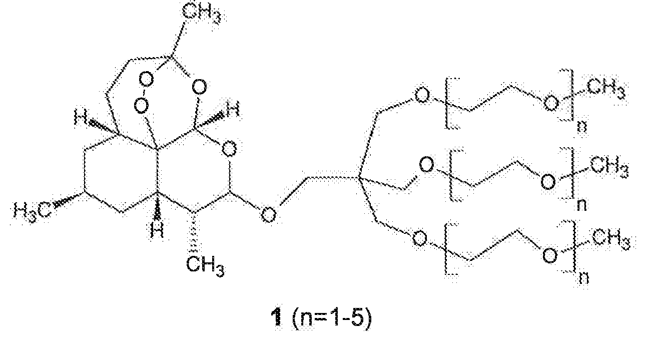

The series of compounds **1**, i.e. all compounds with formula 1 as shown above wherein n is 1, 2, 3, 4 or 5, respectively, have been found to have favorable properties as antimalarials. Crucial to the invention is that the molecule is essentially free from molecules where any of the chain lengths, n, have a different value than the desired one, i.e. the product is of defined molecular weight or, synonymously, monodisperse; so that the purity of the product is more than 70%. In some embodiments the purity of the product is more than 80%. In some embodiments the purity of the product is more than 90%. In some embodiments the purity of the product is more than 95%. In some embodiments the purity of the product is more than 99%.Less advantageous, it can, of course, be envisioned to use unsymmetrical products where the three n:s in the compound would be different e.g. n = 5, 5 and 4 reseptively or n = 5, 4 and 4, respectively,

Also compunds wherein the methyl group at the end of the PEG chains is substituted for another chemical group like hydrocarbon residues, carbonyl derivatives, or heterocyclic groups can be contemplated.

One advantage of the series of compounds **1** is the very high water solubility. These compounds are soluble in water above 1:1 weight:weight. The viscosity of such a solution is approximately 20 mPas which is acceptable for administration via a syringe.

For comparative purposes it might be mentioned that the corresponding linear structure, **2** with n=11, where we have added a solubilizing polyether of similar molecular weight but lacking the branched structure of the present invention, has a similar solubility but the viscosity is in this case unacceptably high (30 mPas). Further, for comparative purposes it is interesting to note that artesunate has a solubility in water of 565 mg/l, almost a factor 2000 less than for a compound of formula **1** wherein n=4.

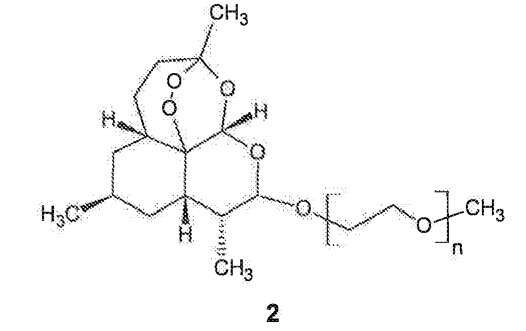

Another advantage of the series of compounds **1** is their surprisingly good acid stability. In this context it is of interest to note that the currently used generation of artemisinin derivatives, in particular artesunate, are sensitive to acid, which lowers the bioavailability after passing through the stomach and also shortens the shelf life of the formulated product. After 72 hours at pH 2.3 artesunate (d) has been degraded to 41 %, compound 2 with n=11 to 40 % and and the compound of formula **1** wherein n=4 to 3%.

The presently disclosed series of compounds **1** are active against *Plasmodium falciparum in-vitro.* It is demonstrated in Example 14 below that a compound of formula 1 wherein n=4 kills half of the parasites *Plasmodium falciparum in-vitro* at a concentration of 43 nM (IC₅₀) whereas it is necessary to use 83 nM of the non-branched compound of formula 2 wherein n=11 to kill half the parasites.

Compounds according to formula 1 also have the advantage of being economically viable. The price of the polyether alcohols of defined molecular weight that serve as starting material for the compounds of formula **1** of the present invention rises very sharply with molecular weight. Especially compounds with formula 1 wherein n = 2, 3 or 4 give a good compromise between cost and performance.

In some embodiments n=1.

In some embodiments n=2.

In some embodiments n=3.

In some embodiments n=4.

In some embodiments n=5.

### Second aspect - A process to produce compounds with formula 1

A second aspect of the present invention relates to the process of producing molecules according to the first aspect. Products 1 are possible to produce by several routes obvious to the one skilled in the art but the process that is described here as a second aspect of this invention has the advantage of using a cheap commercially available trihaloalcohol as a key intermediate and source of the quaternary carbon as outlined in schemes 1 and 2 below.

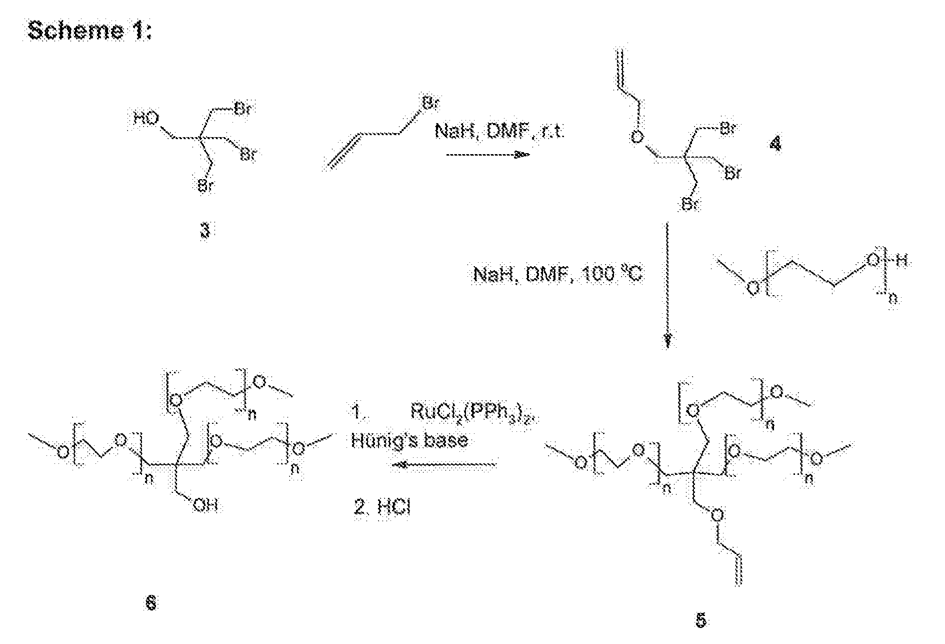

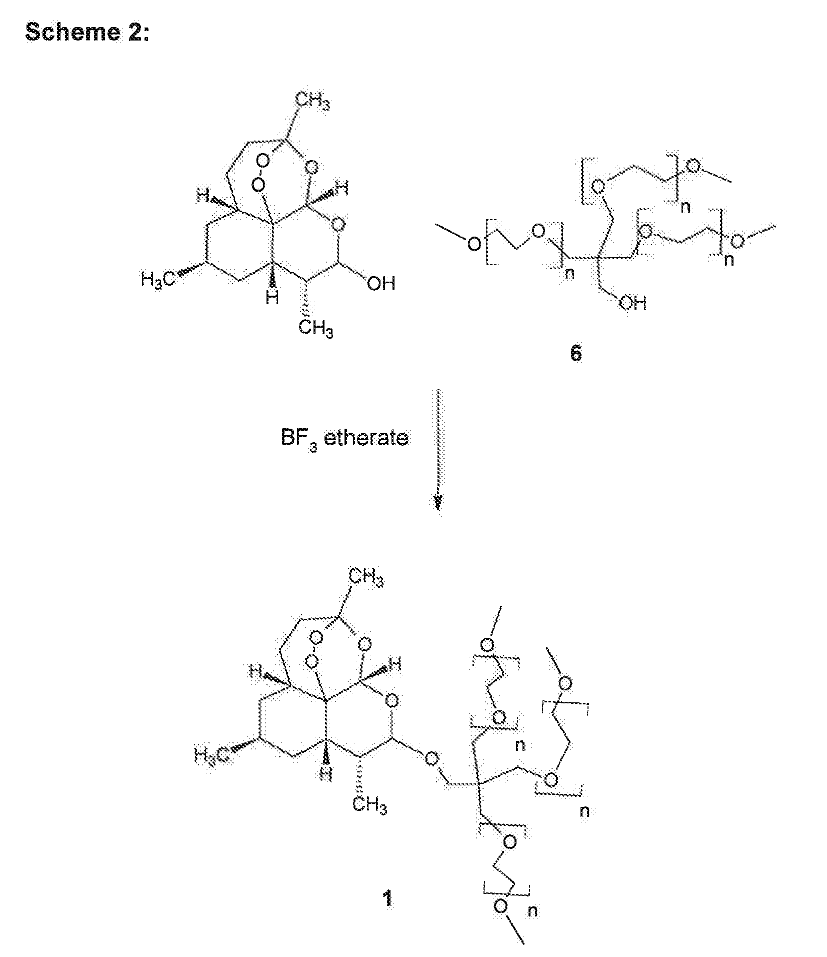

The type of artemisinin derivatives disclosed herein, i.e. compounds with formula 1 are readily produced from inexpensive starting materials using a concise synthetic strategy outlined in schemes 1 and 2 above.

Depending on the origin of the polyether alcohol used in the synthesis of the branched derivatives of the present invention, the final product will have a spectrum of minor impurities. Some of those impurities may consist of compounds having general formula **1** but wherein not all n:s are equal, i.e. 1, 2, 3, 4 or 5, but rather a combination of two or more of these values, and these compounds are thus not desired compounds but considered as impurities.

In general, it is advisable to use pure starting materials. Suitable purities of the starting materials are higher than 70% or higher than 90% or higher than 95% or higher than 99%.

There may also be residues from incomplete reaction of the intermediates which can be minimized on a case by case basis by optimizing reaction time, reaction temperature, amount of solvent, identity of the solvent, or identity of the base.

The process for the production of a compound of formula **1** comprises the following steps:
i. contacting a solution of (3-halo-2,2-bis(halomethyl)propanol) in an aprotic solvent is with CH₃-(OCH₂CH₂)ₙO⁻; or base and CH₃(OCH₂CH₂)ₙOH wherein n=1-5,
ii. heating the mixture from step i at a temperature of 30-150 °C, such as 70-120 °C or 90-110 °C, for a duration of between 30 minutes and 30 hours,
iii. the product from step ii is added to a solution of dihydroartemisinin and a Lewis acid, and
iv. isolating the compound from the mixture obtained in step iii.

In more detail, the process for producing compounds of formula **1** comprises the steps of:
a. Contacting the key starting material 3-halo-2,2-bis(halomethyl)propanol, preferably in the form of 3-bromo-2,2-bis(bromomethyl)propanol (denoted **3** in Scheme 1),with an allylation reagent such as, allyl chloride, allyl me-sylate, or allyl tosylate or, preferrably, allyl bromide, optionally in a solvent inert to the reaction conditions, in the presence of a strong base, for example sodium hydride. The intermediate (denoted **4** in Scheme 1) may then be isolated by any standard technique described in organic chemsitry textbooks (Advanced practical organic chemistry, Leonard, Lygo and Procter 1998, 2nd ed, Stanley Thornes Publishers, Cheltenham). It is also conceivable to proceed with the next step without isolating the intermediate 3-(3-bromo-2,2-bis(bromomethyl)propoxy)prop-1-ene.
b. Contacting the intermediate product with a PEG-oligomer of structure CH₃-(OCH₂CH₂)ₙO⁻ ; or CH₃-(OCH₂CH₂)ₙOH wherein n=1-5, with a defined molecular weight, optionally in a solvent inert to the reaction conditions, in the presence of a strong base, such as sodium hydride. The temperature in this step may advantageously be above room temperature such as between 30 and 150 °C or between 70 and 120 °C or preferably between 90 and 110 °C. Optionally the resulting branched PEG intermediate (denoted **5** in Scheme 1) may be isolated by any standard technique described in organic chemistry textbooks (Advanced practical organic chemistry, Leonard, Lygo and Procter 1998, 2nd ed, Stanley Thornes Publishers, Cheltenham).
c. The intermediate **5** of step b is then transformed into the polyether alcohol (denoted **6** in Scheme 1)by any of the many methods obvious to one skilled in the art. Some not limiting examples are discussed below and further elaborated in examples 3-6.
d. Contacting the intermediate alcohol **6** with dihydroartemisinin or an equally activated analog of dihydroartemisinin in the presence of a Lewis acid to produce compound **1**, as shown in Sceme 2.

The word "activated analog " above refers to dihydroartemisinin derivatives where the hydroxy group has been substituted to an ether, a thioether, a halogen, a sulfonate, a phosphate, a phosphonate, a heterocycle or a carboxyl derivative .

In some embodiments it is favourable to use an extractive procedure or vacuum distillation in step a.

In some embodiments it is favourable to use an extractive procedure followed by a simple chromatography in step b.

In some embodiments the allyl group used in step a is removed by the use of KOt-Bu (Potassium tert-butoxide) in DMSO, followed by HCl (hydrochloric acid).

In some embodiments the Lewis acid used in step d is borontrifluoride or borontrifluoride ethereate.

### Third aspect - Medical use of the compounds of formula 1

In a third aspect of the present invention, at least one of the compounds of formula **1** are used in the treatment or prophylaxis of a disease, in particular, a parasitic disease. In preferred embodiments the parasitic disease is malaria, caused by the protist family *Plasmodium,* in particular *P. falcipartum, P. vivax, P.ovale* or *P. malariae.*

The treatment is carried out by administering a pharmaceutically acceptable formulation comprising at least one compound of formula **1** to a patient in need of such treatment in one or more doses.

In some embodiments of the invention, a compound of formula **1** is administered in combination with one or more other drugs such as antimalarial drugs. In some embodiment at least one of the other drugs is selected from the group consisting of lumefantrine, quinine, or choroquine, amodiaquine, tetracycline, doxycycline, mefloquine and primoquine. In some embodiments a compound of formula **1** is administered together with sulfadoxime and pyrimethamine, optionally together with one or more additonal, other drugs.

The compounds of formula **1** may be administered as separate tablets, capsules, solution for injection or any other pharmaceutically acceptable formulation together with one or more separate or combined antimalarial drugs.

The compounds of formula **1** may also be administered as a combination formulation with one or more of said antimalarial drugs.

Many routes of administration are known in the art and depending on the condition of the patient any of the following routes of administration may be relevant: oral, sublingual, buccal, nasal, inhalatory, transdermal, parenteral (including intraperitoneal, intraorgan, subcutaneous, intradermal, intramuscular, intraarticular, venous, lymphatic, cardiac, arterial, via catheter into the brain parenchyma or ventricles), enteral including, rectal, vaginal, urethral or bladder cisternal.

### Fourth aspect - Pharmaceutically acceptable formulations comprising a compound of formula 1

In a fourth aspect a compound of formula **1** is included in pharmaceutical acceptable formulation or composition. The formulation or composition may contain any pharmaceutically acceptable compounds with the purpose of enhancing the properties of the formulation or composition. Typical but not limiting properties that may be improved by additives to the formulation is pH, stability, taste, smell, color, powder flow properties, hygroscopicity, viscosity, tablet formation, tablet press release, tablet dissolution, shelf-life, or oxidation resistance. A current but non-limiting list of acceptable additives can be found in handbook of pharmaceutical excipients (Pharmaceutical Press; 6th ed 2009). Advantageously, the formulation or composition may contain one or more other pharmacologically active compounds. In one aspect, one or more of the pharmacologically active components is active against a parasitic disease such as malaria, alone or synergistically in combination with a compound of formula **1** or yet another of the components. In some embodiments at least one of the other drugs included in the formulation or composition is selected from the group consisting of lumefantrine, quinine, choroquine, amodiaquine, tetracycline, doxycycline, mefloquine and primoquine. In some embodiments a compound of formula 1 is formulated together with sulfadoxime and pyrimethamine, optionally in combination with yet one or more other drugs.

The formulation may, as non-limiting examples, take the form of tablets, lozenges, capsules, suppositories, suspensions, solutions, emulsions, syrups, granulates, or powders.

In the case where a formulation or composition comprising a compound of formula **1** is given together with one or more other drugs the tables or capsules may be packed in blister packs with the two or more drugs in separate rows, facilitating patient compliance with the treatment scheme.

Some embodiments are solutions comprising a compound of formula **1** alone or in combination with another drug such as said antimalarial drugs, suitable for parenteral admistration.

Some embodiments are freeze dried sterile products comprising a compound of formula **1** alone or in combination with another drug such as said antimalarial drugs, suitable for reconstitution with sterile water or saline.

Some embodiments are solutions comprising a compound of formula **1** alone or in combination with another drug such as said antimalarial drugs, suitable for intravenous injection.

Some embodiments are solutions comprising a compound of formula **1** alone or in combination with another drug such as said antimalarial drugs, suitable for oral administration.

Some embodiments are tablets comprising a compound of formula **1** alone or in combination with another drug such as said antimalarial drugs, suitable for oral administration.

Some embodiments are suppositories suitable for rectal administration comprising a compound of formula **1** alone or in combination with another drug such as said antimalarial drugs,.

### Brief description of the drawing

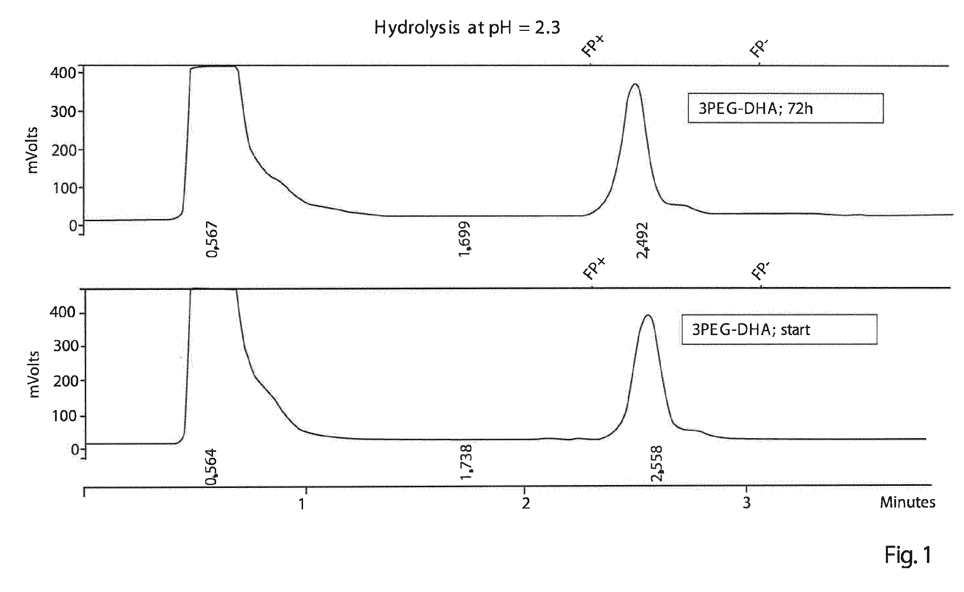

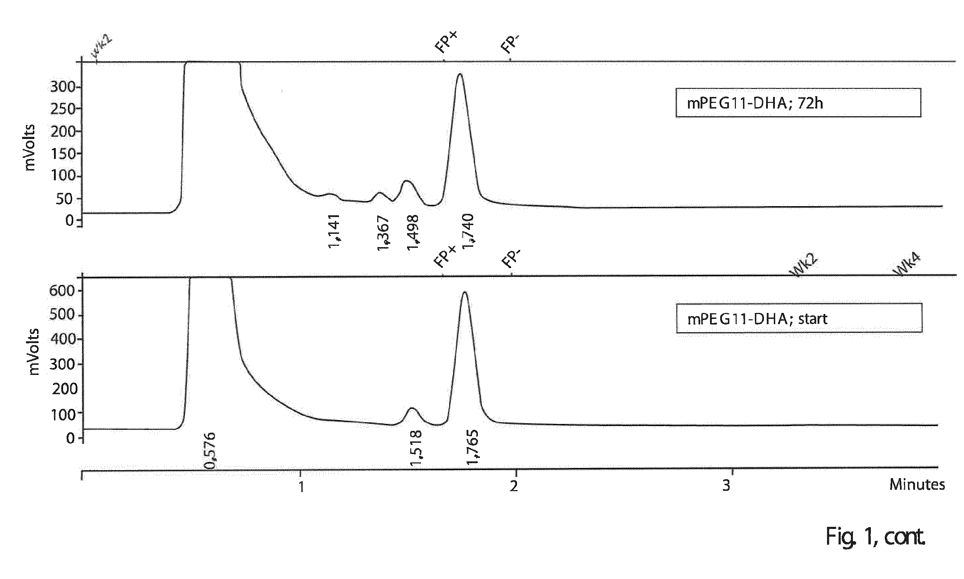

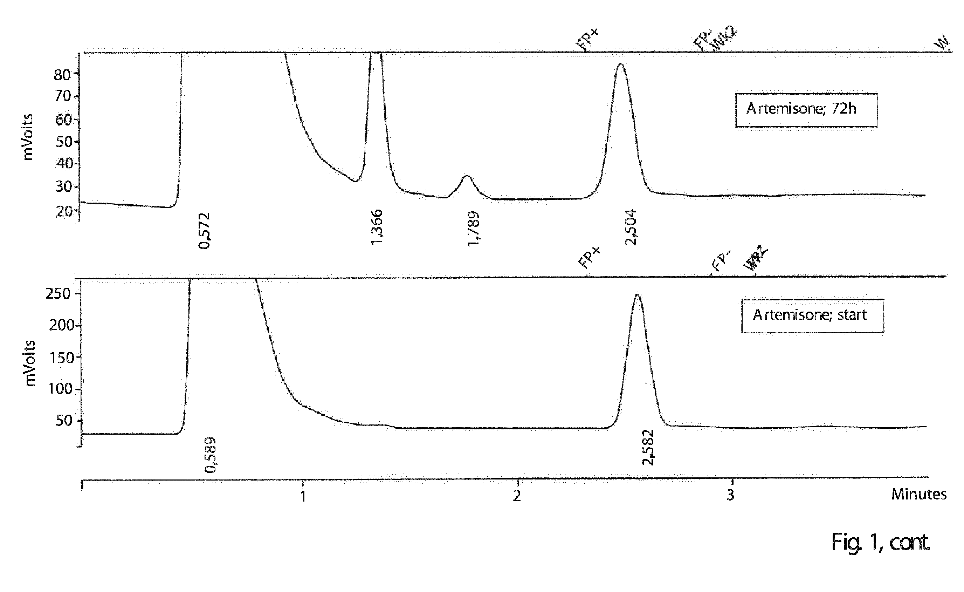

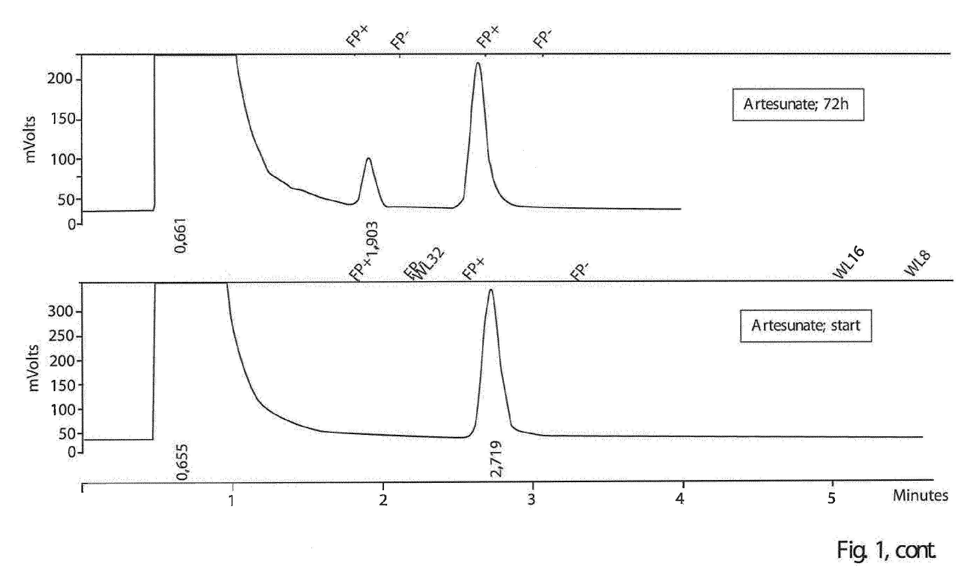

In Example 11 below reference is made to the appended drawing on which Figure 1 shows the result of acid stability testing for four dihydroartemisinin (DHA) derivatives, more precisely for the compound of formula 1 with n=4 (3PEG-DHA) dissolved in H₂O to give 0.48 mg/ml in 0.1 M buffer, the compound of formula 2 with n=11 (mPEG11-DHA) dissolved in H₂O to give 0.42 mg/ml in 0.1 M buffer, artemisone dissolved in mob. phase to give 0.20 mg/ml in 0.1 M buffer and artesunate dissolved in mob. phase to give 0.24 mg/ml in 0.1 M buffer.

### Examples

### Example 1. 3-(3-Bromo-2,2-bis(bromomethyl)propoxy)prop-1-ene (compound 4 of Scheme 1).

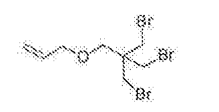

Sodium hydride (1.68 g, 42 mmol) was added in portions to to 3-bromo-2,2-bis(bromomethyl)propanol (9.75g, 30 mmol) and allyl bromide (12.9 ml, 150 mmol) in dry and degassed (by vacuum) DMF (40 ml, dried over 4A molecular sieves for 24h) under nitrogen at 0 °C. The temperature was then increased to room temperature (22 °C) and the reaction mixture was stirred for another 3 h. It was then slowly added to aqueous, saturated NH₄Cl (50 ml). The H₂O-phase was then extracted with diethyl ether (2 x 50 ml) and the combined organic phases were washed with H₂O (5 x 50 ml) and then brine (50 ml). The organic phase was dried with Na₂SO₄ followed by filtration. The volatile materials were removed at reduced pressure to give a pale yellow oil (9.7 g). Column chromatography (heptane:EtOAc 9:1) gave 6.6 g (62 %) of compound **4** as a clear oil. The product could also be isolated using distillation at reduced pressure, boiling point 94-96 °C at 0.04 mbar. ¹H-NMR (CDCl₃); 5.93 (m, 1H), 5.28 (m, 2H), 4.05 (d, 2H), 3.58 (s, 6H), 3.52 (s, 2H).

### Example 2. Allyl 2,2-bis(2,5,8,11-tetraoxadodecyl)-4,7,10,13-tetraoxatetradecyl ether (compound 5 of Scheme 1 wherien n=3)

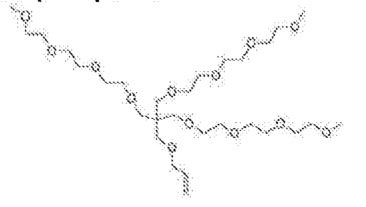

Triethyleneglycol monomethyl ether (4.5 ml, 31 mmol) dissolved in dry and degassed DMF (10 ml) was carefully added to sodium hydride (1.3 g, 31 mmol) in dry and degassed DMF (30 ml) under nitrogen at 0 °C using a syringe. The temperature was then raised to room temperature and the reaction mixture was stirred for another 30 min. Tribromide (2.55 g, 7.0 mmol, example 1) was then added and the temperature was raised to 100 °C. After 18 h the reaction was completed and the temperature was decreased to room temperature whereupon the reaction mixture was slowly added to brine (40 ml). The H₂O-phase was extracted with EtOAc (3 x 40 ml) and the combined organic phases were washed with brine (2 x 30 ml). Sodium sulfate and charcoal was added to the organic phase. The clear organic phase was filtered and the volatile material was removed at reduced pressure (8 mmHg, 40 °C then 0.1 mmHg (oil pump) and 40 °C to remove residual DMF). Column chromatography (EtOAc:THF 8:1) gave 2.1 g of the product. ¹H-NMR (CDCl₃); 5.85 (m, 1H), 5.18 (m, 2H), 3.95 (d, 2H), 3.70-3.55 (m, 36H), 3.43 (s, 6H), 3.40 (s, 2H), 3.38 (s, 9H).

### Example 3. Allyl 2,2-bis(2,5,8,11,14-pentaoxadodecyl)-4,7,10,13,16-pentaoxatetradecyl ether (compound 5 of Scheme 1 wherein n=4)

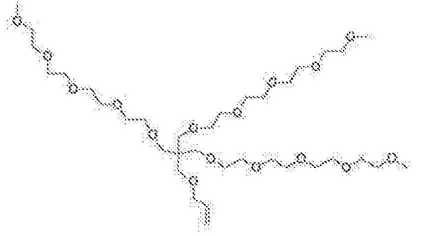

Tetraethyleneglycol monomethyl ether (1.91 ml, 9 mmol) dissolved in dry and degassed DMF (3.5 ml) was carefully added to sodium hydride (365 mg, 9 mmol) in dry and degassed DMF (15 ml) under nitrogen at 0 °C using a syringe. The temperature was then raised to room temperature and the reaction mixture was stirred for another 30 min. Tribromide **4** (730 mg, 2.0 mmol, example 1) was then added and the temperature was raised to 100 °C, After 14 h the reaction was completed and the temperature was decreased to room temperature whereupon the reaction mixture was slowly added to H₂O (150 ml). The H₂O-phase was washed with diethyl ether (2 x 50 ml). Sodium chloride was then added to the H₂O-phase until saturation. The H₂O-phase was extracted with EtOAc (4 x 50 ml) and the combined organic phases were washed with brine (2 x 30 ml). Sodium sulfate and charcoal was added to the organic phase. The clear organic phase was filtered and the volatile material was removed at reduced pressure. Column chromatography (EtOAc:MeOH 9:1) gave 1.05 g of the product. ¹H-NMR (CDCl₃); 5.90 (m, 1H), 5.20 (m, 2H), 3.94 (dt, 2H), 3.70-3.55 (m, 48H), 3.45 (s, 6H), 3.43 (s, 2H), 3.40 (s, 9H).

### Example 4. Allyl 2,2-bis(2,5,8,11-tetraoxadodecyl)-4,7,10,13-tetraoxatetradecanol (compound 6 of Scheme 1 wherein n=3)

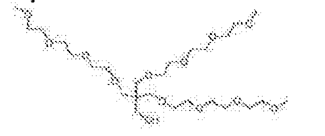

Dichlorotris(triphenylphosphine)ruthenium (155 mg, 0.16 mmol) and diisopropylethylamine (0.56 ml, 3.2 mmol) was added to **5**, n=3 (0.99 g, 1.6 mmol, example 2) in dry toluene (16 ml) under nitrogen. The reaction mixture was stirred at 110 °C for 16 h. The temperature was lowered and volatile components were removed at reduced pressure. H₂O (13 ml) was then added to the residue and the mixture was shaken vigorously for 2 min. The mixture was then filtered and the filtrate was concentrated at reduced pressure. Toluene was added to the residue and then removed at reduced pressure. This procedure was repeated twice. The residue was dried using an oil pump. The crude product from previous procedure was dissolved in aqueous HCl (13 ml, 0.1M). The reaction mixture was shaken at 60 °C for 90 min. H₂O was removed at reduced pressure. Toluene was added to the residue and then removed at reduced pressure. This procedure was repeated twice. The residue was dried using an oil pump and purified by column chromatography (DCM with 5% MeOH) to give 0.93 g of the product as a light yellow oil. ¹H-NMR (CDCl₃); 3.66-3.53 (m, 38H), 3.47 (s, 6H), 3.34 (s, 9H).

### Example 5. Allyl 2,2-bis(2,5,8,11,14-pentaoxadodecyl)-4,7,10,13,16-pentaoxatetradecanol (compound 6 of Scheme 1 wherein n=4)

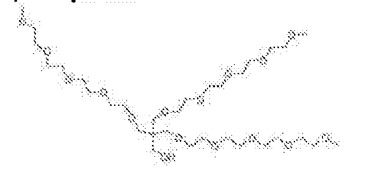

Dichlorotris(triphenylphosphine)ruthenium (288 mg, 0.3 mmol) and diisopropylethylamine (1.04 ml, 6 mmol) was added to 5, n=4 (2.24 g, 3 mmol, example 3) in dry toluene (30 ml) under nitrogen. The reaction mixture was stirred at 110 °C for 80 min. The temperature was lowered and volatile components were removed at reduced pressure. H₂O (25 ml) was then added to the residue and the mixture was shaken vigorously for 2 min. The mixture was then filtered and the filtrate was concentrated at reduced pressure. Toluene was added to the residue and then removed at reduced pressure. This procedure was repeated twice. The residue was dried using an oil pump. The crude product was dissolved in aqueous HCl (20 ml, 0.1M). The reaction mixture was shaken at 60 °C for 90 min. H₂O was removed at reduced pressure. Toluene was added to the residue and then removed at reduced pressure. This procedure was repeated twice. The residue was dried using an oil pump to give 1.5 g of the product as a light brown oil. ¹H-NMR (CDCl₃); 3.66-3.52 (m, 50H), 3.47 (s, 6H), 3.37 (s, 9H).

### Example 6. Compound of formula 1 wherein n=3

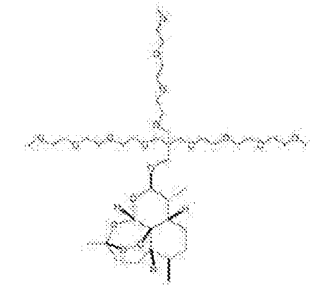

Bortrifluoride etherate (84 µl, 0.68 mmol) was added to **6**, n=3 (391 mg, 0.68 mmol, example 4) and dihydroartemisinin (386 mg, 1.36 mmol) in dry diethyl ether (16 ml) at room temperature. After 4 h, more bortrifluoride etherate (21 µl, 0.17 mmol) was added and the reaction mixture was stirred for another 20 h. Dilute aqueous sodium bicarbonate (2%) was added and the reaction mixture was stirred for another 30 min. The phases were separated and the aqueous phase was saturated with NaCl (s) and then extracted with ethyl acetate (3 x 30 ml). The combined organic phases were dried over sodium sulphate. Filtration and removal of the solvent at reduced pressure followed by column chromatography (EtOAc:THF 4:1) gave 110 mg of the product as a mixture of α and β isomer. HPLC analysis (HPLC-ELSD-C18, 75:25 to 5:95 H2O/ACN in 20 min) displayed the product peak at 9 min. ¹H-NMR (CDCl₃); 5.42 (s, 1H, a); 5.38 (s, 1H, b); 4.84 (d, 1H, b); 4.75 (d, 1H, a); 3.90 (d, 1H); 3.68-3.53 (m, 36 H); 3.43 (s, 6H); 3.40 (s, 9H); 3.31 (d, 1H); 2.63 (m, 1H); 2.38 (m, 1H); 2.10-0.90 (m, 10H); 1.44 (s, 3H); 0.97 (d, 3H); 0.90 (d, 3H)

### Example 7. Compound of formular 1 wherein n=4.

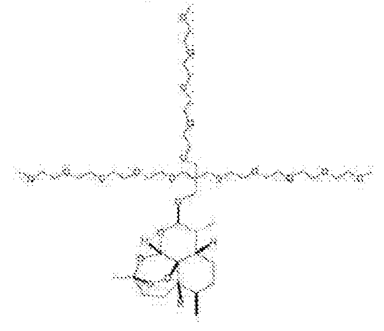

Bortrifluoride etherate (124 µl, 1.0 mmol) was added to 6, n=4 (707 mg, 1.0 mmol, example 5) and dihydroartemisinin (426 mg, 1.5 mmol) in dry diethyl ether (20 ml) at room temperature. After 4 h, more bortrifluoride etherate (31 µl, 0.25 mmol) was added and the reaction mixture was stirred for another 20 h. Dilute aqueous sodium bicarbonate (2%) was added and the reaction mixture was stirred for another 30 min. The phases were separated and the aqueous phase was saturated with NaCl (s) and then extracted with ethyl acetate (3 x 30 ml). The combined organic phases were dried over sodium sulphate. Filtration and removal of the solvent at reduced pressure followed by column chromatography (DCM to DCM/MeOH 95:5) gave 780 mg of the product as a mixture of α and β isomer, HPLC analysis (HPLC-ELSD-C18, 40:60 to 5:95 H2O/ACN in 20 min) displayed the product peak at 13 min. MS (ESP+) [M+Na+]: 995.6. ¹H-NMR (acetone-d₆); 5.41 (s, 1H, α); 5.40 (s, 1H, β); 4.67 (d, 1H, β); 4.48 (d, 1H, α); 3.85 (d, 1H); 3.68-3.43 (m, 54 H); 3.31 (s, 9H); 3.27 (d, 1H); 2.54 (m, 1H); 2.29 (m, 1H); 2.10-0.90 (m, 10H); 1.32 (s, 3H); 0.97 (d, 3H); 0.94 (d, 3H)

### Example 8. Compound of formula 2 wherein n=11

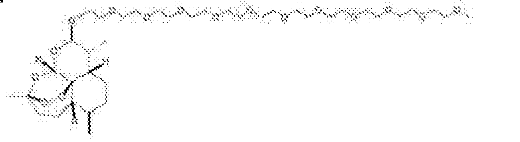

Bortrifluoride etherate (62 µl, 0.5 mmol) was added to mPEG(11)-OH (516 mg, 1.0 mmol) and dihydroartemisinin (426 mg, 1.5 mmol) in dry diethyl ether (20 ml) at room temperature. After 2 h, more bortrifluoride etherate (124 µl, 1.0 mmol) was added and the reaction mixture was stirred for another 20 h. Dilute aqueous sodium bicarbonate (2%) was added and the reaction mixture was stirred for another 30 min. The phases were separated and the aqueous phase was saturated with NaCl (s) and then extracted with ethyl acetate (3 x 30 ml). The combined organic phases were dried over sodium sulphate. Filtration and removal of the solvent at reduced pressure followed by column chromatography (DCM to DCM/MeOH 95:5) gave 300 mg of the product as a mixture of α and β isomer. HPLC analysis (HPLC-ELSD-C18, 40:60 H2O/ACN) displayed the product peak at 11 min. ¹H-NMR (acetone-d₆); 5.43 (s,1H,b); 5.40 (s, 1H, a); 4.74 (d, 1H, b);4.58 (d, 1H, a); 3.88 (d, 1H); 3.70-3.43 (m, 43 H); 3.29 (s, 3H); 2.50 (m, 1H); 2.27 (m, 1H); 2.10-0.90 (m, 10H); 1.30 (s, 3H); 0.95 (d, 3H); 0.91 (d, 3H)

### Example 9. Compound of formula 2 wherein n=7.

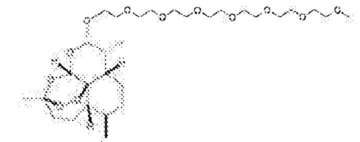

Bortrifluoride etherate (124 µl, 1.0 mmol) was added to mPEG(7)-OH (340 mg, 1.0 mmol) and dihydroartemisinin (426 mg, 1.5 mmol) in dry diethyl ether (20 ml) at room temperature. After 4 h, more bortrifluoride etherate (31 µl, 0.25 mmol) was added and the reaction mixture was stirred for another 20 h. Dilute aqueous sodium bicarbonate (2%) was added and the reaction mixture was stirred for another 30 min. The phases were separated and the aqueous phase was saturated with NaCl (s) and then extracted with ethyl acetate (3 x 30 ml). The combined organic phases were dried over sodium sulphate. Filtration and removal of the solvent at reduced pressure followed by column chromatography (EtOAc:THF 4:1) gave 276 mg of the product as a single isomer. HPLC analysis (HPLC-ELSD-C18, 90:10 to 5:95 H2O/ACN in 20 min) displayed the product peak at 21 min. H-NMR (CDCl₃)_{;} 5.42 (s, 1H); 4.82 (d, 1H); 3.91 (d, 1H); 3.70-3.50 (m, 27 H); 3.36 (s, 3H); 2.60 (m, 1H); 2.35 (m, 1H); 2.10-0.90 (m, 10H); 1.42 (s, 3H); 0.95 (d, 3H); 0.90 (d, 3H)

### Example 10. Compound of formula 2 wherein n=5

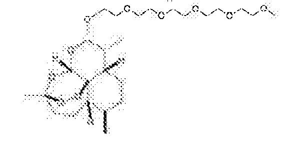

Bortrifluoride etherate (124 µl, 1.0 mmol) was added to pentaethyleneglycol monomethyl ether (252 mg, 1.0 mmol) and dihydroartemisinin (426 mg, 1.5 mmol) in dry diethyl ether (20 ml) at room temperature. After 4 h, more bortrifluoride etherate (31 µl, 0.25 mmol) was added and the reaction mixture was stirred for another 20 h. Dilute aqueous sodium bicarbonate (2%) was added and the reaction mixture was stirred for another 30 min. The phases were separated and the aqueous phase was saturated with NaCl (s) and then extracted with ethyl acetate (3 x 30 ml). The combined organic phases were dried over sodium sulphate. Filtration and removal of the solvent at reduced pressure followed by column chromatography (EtOAc) gave 200 mg of the product as a single isomer. ¹H-NMR (CDCl₃); 5.42 (s, 1H); 4.82 (d, 1H); 3.93 (d, 1H); 3.70-3.53 (m, 19 H); 3.38 (s, 3H); 2.61 (m, 1H); 2.37 (m, 1H); 2.10-0.90 (m, 10H); 1.42 (s, 3H); 0.95 (d, 3H); 0.90 (d, 3H).

### Example 11. The acid stability of four dihydroartemisinin (DHA) derivatives was investigated.

The compound of formula 1 wherien n=4, the compound of formula **2** wherein n=11, artemison and artesunate were treated with ammoniumacetate buffer pH = 5.0 and Sodiumphosphate pH =2.3
The stability of the compounds was followed with HPLC.

### Experimental:

### Column:

Phenomex Luna; 50 x 4.6 mm, 3 µ C18.

### Mob. phase:

ACN/H₂O; 60/40 at 1.0 ml/min

### Sample preparation:

DHA dissolved in mob. phase to give 0,21 mg/ml

Compound of formula 1, n=4 (3PEG-DHA) dissolved in H₂O to give 0.48 mg/ml in 0.1 M buffer.

Compound of formula 2, n=11 (mPEG11-DHA) dissolved in H₂O to give 0.42 mg/ml in 0.1 M buffer.

Artemisone dissolved in mob. phase to give 0.20 mg/ml in 0.1 M buffer,

Artesunate dissolved in mob. phase to give 0.24 mg/ml in 0.1 M buffer.

### Injection:

20 µl

### Detection:

ELSD; 45°C, 1.2 l/min

### Results (illustrated in Fig. 1):

No sign of degradation at pH = 5.0 of any of the samples (3PEG-DHA, mPEG11-DHA, artemison and artesunate) after 21 h (results not shown in the figure).

After 72h at pH = 2.3 the degradation was more pronounced.

For 3PEG-DHA the area of the main peak has decreased about 3 % .

For mPEG11-DHA the area of the main peak has decreased about 45 % and DHA also appear in the chromatogram.

For artemison the area of the main peak has decreased about 71 % and DHA also appear in the chromatogram.

For artesunate the area of the main peak has decreased about 40 % and DHA also appear in the chromatogram. However the chromatographic conditions were changed to 50 % ACN/ 75 mM HCOO(NH₄); pH=3.75 in order to better separate artesunate from "salt peaks".

### Example 13. Viscosity

The viscosity of a solution of the compound of formula **1** wherein n=4, at a concentration of 95,9 mg / 99,0 mg water was found to be 20 mPas at room temperature as measured in a provisional set up by flow time through a syringe needle. Water has a viscosity of about one mPas and the maximum viscosity that is easily injected manually is about 25 mPas. The corresponding linear PEG-ylated artemisinin, SI042, has a non-acceptable viscosity of 31 mPas at the same concentration.

### Example 14. Biological activity

The effect on *Plasmodium falciparum* was determined by quantifying the effect of the compounds under 72 hours (1.5 parasite replication cycles) by measuring the amount of histidine rich protein by an ELISA method. Each concentration was measured in triplicate and a concentration range from 0.1 nM to 10 mM was investigated. The IC₅₀ value of the compound of formula **1** wherein n=4 was found to be 43 nm, and for the compound of formula **2** wherein n=11 the IC₅₀ value was 83 nm.

## Claims

1. A compound of general formula **1** 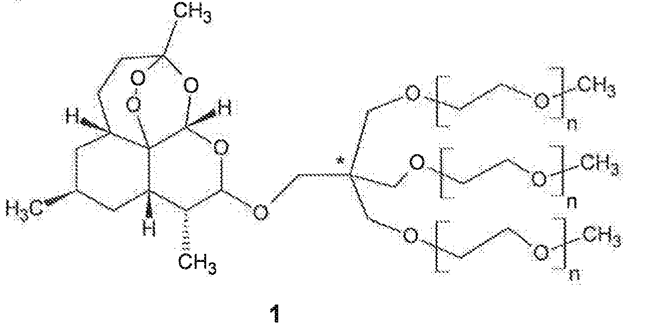 wherein n=1-5.

2. A compound according to claim 1, wherein at least one of the methyl group at the end of the PEG chains is substituted with a hydrocarbon residue, a carbonyl derivative or a heterocyclic group.

3. A compound according to claim 1 or 2, wherein n is 2, 3 or 4.

4. A compound according to any one of the claims 1-3, wherein the carbon atom marked * originates from 3-halo-2,2-bis(halomethyl)propanol.

5. A composition comprising compounds according to any one of the claims 1-3.

6. A composition according to claim 4, wherein said compounds are monodisperse so that the purity of the part of the composition constituted by said comounds is more than 70%.

7. A composition according to claim 5 or 6, wherein said composition is a pharmaceuticily acceptable composition which in addition to the compounds according to any one of the claims 1-3 optionally comprises at least one pharmaceutically acceptable adjuvant or carrier.

8. A pharmaceutically acceptable composition according to claim 7, formulated as a tablet, a capsule, a lozenge, a liquid suitable for oral ingestion, a reconstitutable solid suitable for reconstitution with water or saline to a solution for injection or infusion, or a solution ready for injection or infusion .

9. A pharmaceutical acceptable composition according to claim 7 or 8, further comprising at least one additional drug.

10. A pharmaceutically acceptable composition according to claim 9, wherein an additonal drug is an antimalarial drug.

11. A pharmaceutically acceptable composition according to claim 9 or 10 wherein said additional drug is selected from the group consisting of lumefantrine, quinine, choroquine, amodiaquine, tetracycline, doxycycline, mefloquine, primoquine, sulfadoxime and pyrimethamine.

12. A compound according to any one of the claims 1-4 or a composition according to any one of the claims 7-11 for use in the treatment of malaria.

13. A process for the production of a compound according to any one of the claims 1-4 comprising the following steps:
i. contacting a solution of (3-halo-2,2-bis(halomethyl)propanol ) in an aprotic solvent is with CH₃-(OCH₂CH₂)ₙO⁻ ; or base and CH₃(OCH₂CH₂)ₙOH wherein n=1-5,
ii. heating the mixture from step i at a temperature of 30-150 °C or between 70 and 120 °C or between 90 and 110 °C for a duration of between 30 minutes and 30 hours,
iii. the product from step ii is added to a solution of dihydroartemisinin and a Lewis acid, and
iv. isolating the compound from the mixture obtained in step iii.

14. A process according to claim 13, wherein the compound produced is the compound according to claim 4 and wherein the 3-halo-2,2-bis(halomethyl)propanol used in step i is 3-bromo-2,2-bis(bromomethyl)-propanol.
